(19) Europäisches Patentamt
European Patent Office
Office européen des brevets

(11)  **EP 2 610 638 B1**

(12)  **EUROPEAN PATENT SPECIFICATION**

(45) Date of publication and mention
of the grant of the patent:
**05.08.2020  Bulletin 2020/32**

(51) Int Cl.:
**G01S 15/89** $^{(2006.01)}$        **G01S 7/52** $^{(2006.01)}$

(21) Application number: **12183245.5**

(22) Date of filing: **06.09.2012**

(54)  **Forming vector information based on vector doppler in ultrasound system**

Erzeugung von Vektorinformationen basierend auf dem Vektordoppler in einem Ultraschallsystem

Formation d'informations de vecteur basée sur un doppler vectoriel dans un système à ultrasons

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2011  KR 20110143861**

(43) Date of publication of application:
**03.07.2013  Bulletin 2013/27**

(73) Proprietor: **Samsung Medison Co., Ltd.**
**Gangwon-do 250-870 (KR)**

(72) Inventors:
• **Lee, Han Woo**
**135-851 Seoul (KR)**
• **Kim, Min Woo**
**135-851 Seoul (KR)**
• **Kim, Deok Gon**
**135-851 Seoul (KR)**
• **Kim, Hyoung Jin**
**135-851 Seoul (KR)**

(74) Representative: **Schmid, Wolfgang**
**Lorenz & Kollegen**
**Patentanwälte Partnerschaftsgesellschaft mbB**
**Alte Ulmer Strasse 2**
**89522 Heidenheim (DE)**

(56) References cited:
**US-A- 4 142 412**

• **DUNMIRE B ET AL: "Cross-beam vector Doppler ultrasound for angle-independent velocity measurements", ULTRASOUND IN MEDICINE AND BIOLOGY, NEW YORK, NY, US, vol. 26, no. 8, 1 October 2000 (2000-10-01), pages 1213-1235, XP004295674, ISSN: 0301-5629, DOI: 10.1016/S0301-5629(00)00287-8**
• **VILKOMERSON D ET AL: "Diffractive transducers for angle-independent velocity measurements", ULTRASONICS SYMPOSIUM, 1994. PROCEEDINGS., 1994 IEEE CANNES, FRANCE 1-4 NOV. 1994, NEW YORK, NY, USA,IEEE, US, 31 October 1994 (1994-10-31), page 1677, XP032084659, DOI: 10.1109/ULTSYM.1994.401913 ISBN: 978-0-7803-2012-3**
• **REZA ZAHIRI AZAR ET AL: "2-D high-frame-rate dynamic elastography using delay compensated and angularly compounded motion vectors: Preliminary results", IEEE TRANSACTIONS ON ULTRASONICS, FERROELECTRICS AND FREQUENCY CONTROL, IEEE, US, vol. 57, no. 11, 1 November 2010 (2010-11-01), pages 2421-2436, XP011320225, ISSN: 0885-3010**

**Description**

CROSS-REFERENCE TO RELATED APPLICATIONS

The present application claims priority from Korean Patent Application No. 10-2011-0143861 filed on December 27, 2011.

TECHNICAL FIELD

The present disclosure generally relates to ultrasound systems, and more particularly to forming vector information corresponding to motion (i.e., velocity and direction) of a target object based on a vector Doppler in an ultrasound system.

BACKGROUND

An ultrasound system has become an important and popular diagnostic tool since it has a wide range of applications. Specifically, due to its non-invasive and non-destructive nature, the ultrasound system has been extensively used in the medical profession. Modern high-performance ultrasound systems and techniques are commonly used to produce two-dimensional or three-dimensional ultrasound images of internal features of target objects (e.g., human organs).

The ultrasound system provides ultrasound images of various modes including a brightness mode image representing reflection coefficients of ultrasound signals (i.e., ultrasound echo signals) reflected from a target object of a living body with a two-dimensional image, a Doppler mode image representing velocity of a moving target object with spectral Doppler by using a Doppler effect, a color Doppler mode image representing velocity of the moving target object with colors by using the Doppler effect, an elastic image representing mechanical characteristics of tissues before and after applying compression thereto, etc.

The ultrasound system transmits ultrasound signals to the living body including a moving target object (e.g., blood flow) and receives ultrasound signals (i.e., ultrasound echo signals) from the living body. The ultrasound system further forms the color Doppler mode image representing velocities of the target object with colors based on the ultrasound echo signals. The color Doppler image is used to diagnose disease of a blood vessel, a heart and the like. However, the color Doppler image cannot represent an accurate motion of the target object since the respective colors in the color Doppler image indicate the velocity of the target object, which moves forward in a transmission direction of the ultrasound signals and backward in the transmission direction of the ultrasound signals.

To resolve this problem, vector Doppler methods capable of obtaining motion (i.e., velocity and direction) of the target object are used. A cross beam-based method of the vector Doppler methods acquires velocity components of the target object from at least two different directions, and combines the velocity components to form vector information including two-dimensional or three-dimensional direction information and velocity information.

Reference is made to DUNMIRE B. ET AL: "Cross-beam vector Doppler ultrasound for angle-independent velocity measurements", ULTRASOUND IN MEDICINE AND BIOLOGY, NEW YORK, NY, US, vol. 26, no. 8, 1 October 2000 (2000-10-01), pages 1213 - 1235, XP004295674, ISSN: 0301-5629, DOI: 10.1016/S0301-5629(00)00287-8.

Reference is also made to VILKOMERSON D. ET AL: "Diffractive transducers for angle-independent velocity measurements", ULTRASONICS SYMPOSIUM, 1994. PROCEEDINGS., 1994 IEEE Cannes, France 1-4 Nov. 1994, New York, NY, USA, IEEE, US, 31 October 1994 (1994-10-31), page 1677, XP032084659, DOI: 10.1109/ULTSYM.1994.401913, ISBN: 978-0-7803-2012-3.

Reference is further made to REZA ZAHIRI AZAR ET AL: "2-D high-frame-rate dynamic elastography using delay compensated and angularly compounded motion vectors: Preliminary results", IEEE TRANSACTIONS ON ULTRASONICS, FERROELECTRICS AND FREQUENCY CONTROL, IEEE, US, vol. 57, no. 11, 1 November 2010 (2010-11-01), pages 2421 - 2436, XP011320225, ISSN: 0885-3010.

SUMMARY

There are provided embodiments for transmitting ultrasound signals to a living body including a moving target object in at least one transmission direction, and receiving ultrasound echo signals from the living body in at least one reception direction to form vector information of the target object.

The invention is defined by claim 1. Regarding the method of forming vector information the invention is defined by claim 5.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1 is a block diagram showing an illustrative embodiment of an ultrasound system.
FIG. 2 is a schematic diagram showing an example of a brightness mode image and a region of interest.
FIG. 3 is a block diagram showing an illustrative embodiment of an ultrasound data acquiring unit.
FIGS. 4 to 7 are schematic diagrams showing examples of transmission directions and reception directions.
FIG. 8 is a schematic diagram showing an example of sampling data and pixels of an ultrasound image.
FIGS. 9 to 12 are schematic diagrams showing examples of performing a receiving beam-forming.
FIG. 13 is a schematic diagram showing an example of setting weights.
FIG. 14 is a schematic diagram showing an example of setting a sampling data set.
FIG. 15 is a flow chart showing a process of forming vector information based on a vector Doppler.
FIG. 16 is a schematic diagram showing an example of the transmission directions, the reception directions, the vector information and an over-determined problem.

DETAILED DESCRIPTION

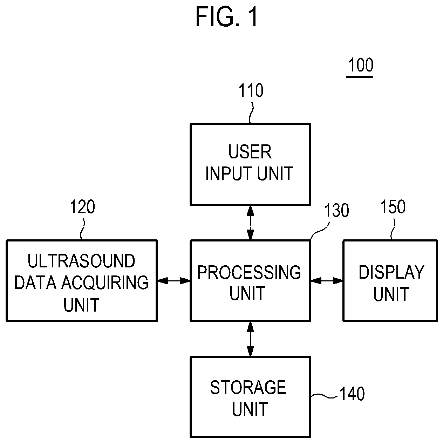

A detailed description is provided with reference to the accompanying drawings. One of ordinary skill in the art should realize that the following description is illustrative only and is not in any way limiting.
Referring to FIG. 1, an ultrasound system 100 in accordance with an illustrative embodiment is shown. As depicted therein, the ultrasound system 100 includes a user input unit 110.

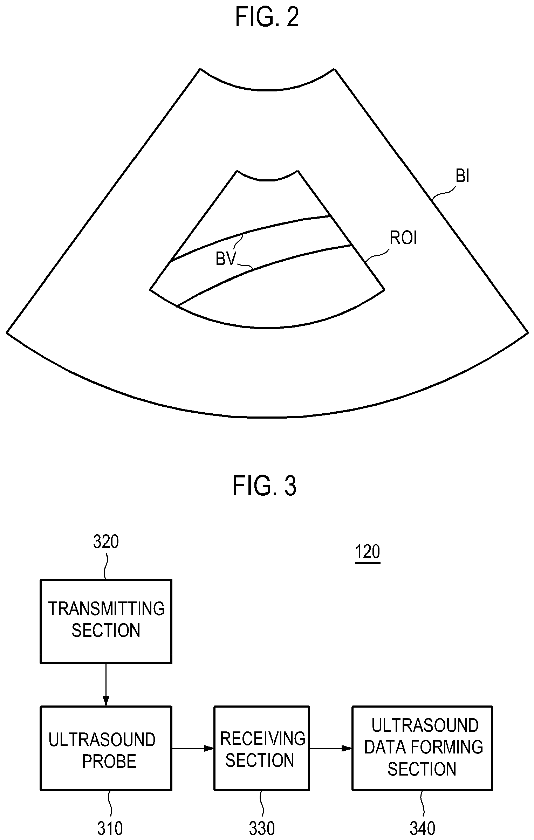

The user input unit 110 is configured to receive input information from a user. The input information includes region of interest setting information for setting a region of interest ROI a brightness mode image BI, as shown in FIG. 2. However, it should be noted herein that the input information may not be limited thereto. The region of interest ROI includes a color box. In FIG. 2, a reference numeral BV represents a blood vessel. The user input unit 110 includes a control panel, a track ball, a mouse, a keyboard and the like.

The ultrasound system 100 further includes an ultrasound data acquiring unit 120. The ultrasound data acquiring unit 120 is configured to transmit ultrasound signals to a living body. The living body includes moving target objects (e.g., blood vessel, heart, blood flow, etc.). The ultrasound data acquiring unit 120 is further configured to receive ultrasound signals (i.e., ultrasound echo signals) from the living body to acquire ultrasound data corresponding to an ultrasound image.

FIG. 3 is a block diagram showing an illustrative embodiment of the ultrasound data acquiring unit. Referring to FIG. 3, the ultrasound data acquiring unit 120 includes an ultrasound probe 310.

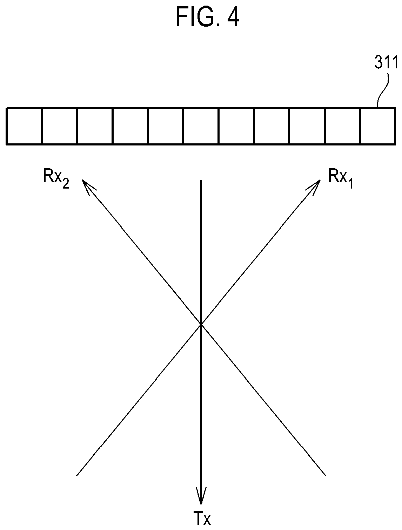

The ultrasound probe 310 includes a plurality of elements 311 (*see* FIG. 4) for reciprocally converting between ultrasound signals and electrical signals. The ultrasound probe 310 is configured to transmit the ultrasound signals to the living body. The ultrasound signals transmitted from the ultrasound probe 310 are plane wave signals that the ultrasound signals are not focused at a focusing point or focused signals that the ultrasound signals are focused at the focusing point. However, it should be noted herein that the ultrasound signals may not be limited thereto. The ultrasound probe 310 is further configured to receive the ultrasound echo signals from the living body to output electrical signals (hereinafter, referred to as "reception signals"). The reception signals are analog signals. The ultrasound probe 310 includes a convex probe, a linear probe and the like.

The ultrasound data acquiring unit 120 also includes a transmitting section 320. The transmitting section 320 is configured to control the transmission of the ultrasound signals. The transmitting section 320 is further configured to generate electrical signals (hereinafter, referred to as "transmission signals") for obtaining an ultrasound image in consideration of the elements 311.

In one embodiment, the transmitting section 320 is configured to generate transmission signals (hereinafter, referred to as "brightness mode transmission signals") for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output reception signals (hereinafter, referred to as "brightness mode reception signals").

The transmitting section 320 is further configured to generate transmission signals (hereinafter, referred to as "Doppler mode transmission signals") corresponding to an ensemble number in consideration of the elements 311 and at least one transmission direction of the ultrasound signals (i.e., transmission beam). Thus, the ultrasound probe 310 is configured to convert the Doppler mode transmission signals provided from the transmitting section 320 into the ultrasound signals, transmit the ultrasound signals to the living body in the at least one transmission direction, and receive the ultrasound echo signals from the living body to output reception signals (hereinafter, referred to as "Doppler mode reception signals"). The ensemble number represents the number of transmitting and receiving the ultrasound signals.

As one example, the transmitting section 320 is configured to generate the Doppler mode transmission signals corresponding to the ensemble number in consideration of a transmission direction Tx and the elements 311, as shown in FIG. 4. The transmission direction Tx is one direction in the range of a direction (0 degree) perpendicular to a longitudinal direction of the elements 311 to a maximum steering direction of the transmission beam.

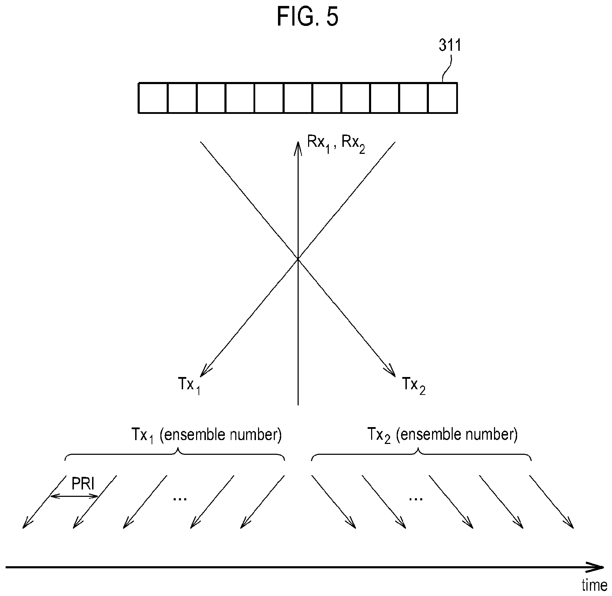

As another example, the transmitting section 320 is configured to generate first Doppler mode transmission signals corresponding to the ensemble number in consideration of a first transmission direction $Tx_1$ and the elements 311, as shown in FIG. 5. Thus, the ultrasound probe 310 is configured to convert the first Doppler mode transmission signals provided from the transmitting section 320 into the ultrasound signals, transmit the ultrasound signals to the living body in the first transmission direction $Tx_1$, and receive the ultrasound echo signals from the living body to output first Doppler mode reception signals. The transmitting section 320 is further configured to generate second Doppler mode transmission signals corresponding to the ensemble number in consideration of a second transmission direction $Tx_2$ and the elements 311, as shown in FIG. 5. Thus, the ultrasound probe 310 is configured to convert the second Doppler mode transmission signals provided from the transmitting section 320 into the ultrasound signals, transmit the ultrasound signals to the living body in the second transmission direction $Tx_2$, and receive the ultrasound echo signals from the living body to output second Doppler mode reception signals. In FIG. 5, a reference numeral PRI represents a pulse repeat interval.

In another embodiment, the transmitting section 320 is configured to generate the brightness mode transmission signals for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output the brightness mode reception signals.

The transmitting section 320 is further configured to generate the Doppler mode transmission signals corresponding to the ensemble number in consideration of the at least one transmission direction and the elements 311. Thus, the ultrasound probe 310 is configured to convert the Doppler mode transmission signals provided from the transmitting section 320 into the ultrasound signals, transmit the ultrasound signals to the living body, and receive the ultrasound echo signals from the living body to output the Doppler mode reception signals. The ultrasound signals are transmitted in an interleaved transmission scheme. The interleaved transmission scheme will be described below in detail.

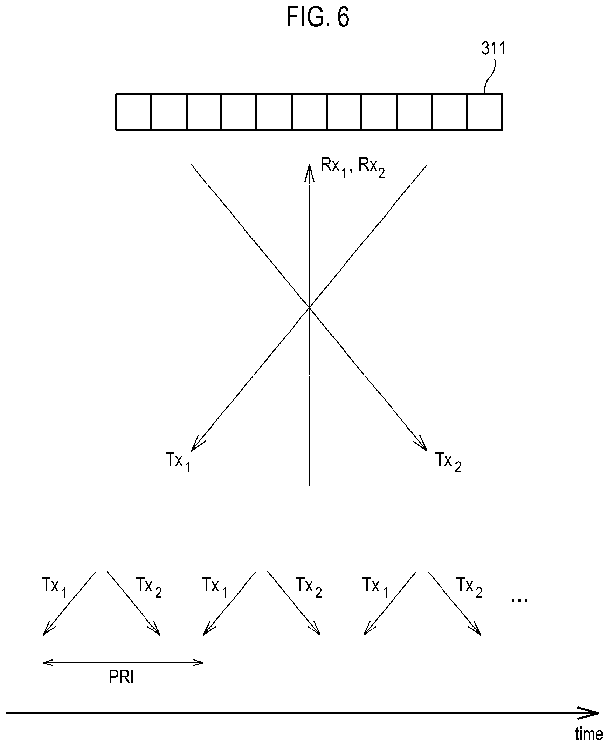

For example, the transmitting section 320 is configured to generate the first Doppler mode transmission signals in consideration of the first transmission direction $Tx_1$ and the elements 311, as shown in FIG. 6. Thus, the ultrasound probe 310 is configured to convert the first Doppler mode transmission signals provided from the transmitting section 320 into the ultrasound signals, and transmit the ultrasound signals to the living body in the first transmission direction $Tx_1$.

Thereafter, the transmitting section 320 is further configured to generate the second Doppler mode transmission signals in consideration of the second transmission direction $Tx_2$ and the elements 311, as shown in FIG. 6. Thus, the ultrasound probe 310 is configured to convert the second Doppler mode transmission signals provided from the transmitting section 320 into the ultrasound signals, and transmit the ultrasound signals to the living body in the second transmission direction $Tx_2$. The ultrasound probe 310 is also configured to receive the ultrasound echo signals (i.e., ultrasound echo signals corresponding to first Doppler mode transmission signals) from the living body to output the first Doppler mode reception signals. The ultrasound probe 310 is further configured to receive the ultrasound echo signals (i.e., ultrasound echo signals corresponding to second Doppler mode transmission signals) from the living body to output the second Doppler mode reception signals.

Thereafter, the transmitting section 320 is configured to generate the first Doppler mode transmission signals based on the pulse repeat interval, as shown in FIG. 6. Thus, the ultrasound probe 310 is configured to convert the first Doppler mode transmission signals provided from the transmitting section 320 into the ultrasound signals, and transmit the ultrasound signals to the living body in the first transmission direction $Tx_1$. The transmitting section 320 is further configured to generate the second Doppler mode transmission signals based on the pulse repeat interval, as shown in FIG. 6. Thus, the ultrasound probe 310 is configured to convert the second Doppler mode transmission signals provided from the transmitting section 320 into the ultrasound signals, and transmit the ultrasound signals to the living body in the second transmission direction $Tx_2$. The ultrasound probe 310 is further configured to receive the ultrasound echo signals (i.e., ultrasound echo signals corresponding to first Doppler mode transmission signals) from the living body to output the first Doppler mode reception signals. The ultrasound probe 310 is also configured to receive the ultrasound echo signals (i.e., ultrasound echo signals corresponding to second Doppler mode transmission signals) from the living body to output the second Doppler mode reception signals.

As described above, the transmitting section 320 is configured to generate the first Doppler mode transmission signals and the second Doppler mode transmission signals corresponding to the ensemble number.

In yet another embodiment, the transmitting section 320 is configured to generate the brightness mode transmission signals for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output the brightness mode reception signals.

The transmitting section 320 is further configured to generate the Doppler mode transmission signals corresponding to the ensemble number in consideration of the at least one transmission direction and the elements 311. Thus, the ultrasound probe 310 is configured to convert the Doppler mode transmission signals provided from the transmitting section 320 into the ultrasound signals, transmit the ultrasound signals to the living body, and receive the ultrasound echo signals from the living body to output the Doppler mode reception signals. The ultrasound signals are transmitted according to the pulse repeat interval.

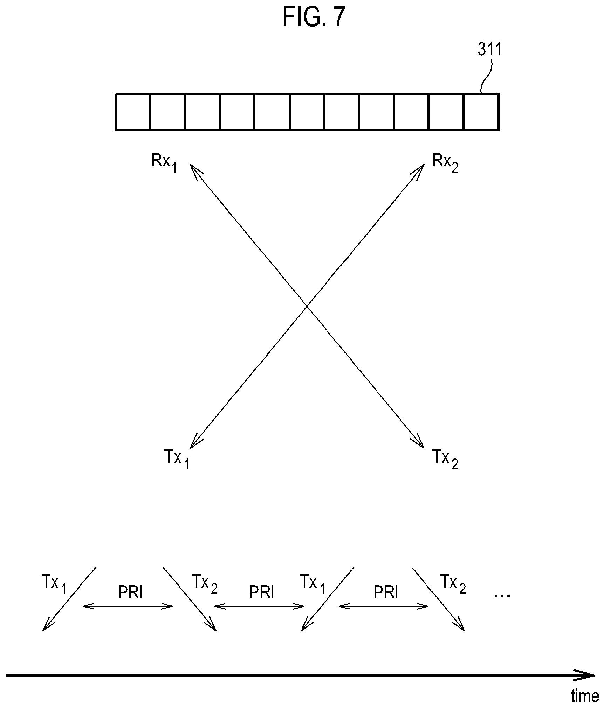

For example, the transmitting section 320 is configured to generate the first Doppler mode transmission signals in consideration of the first transmission direction $Tx_1$ and the elements 311 based on the pulse repeat interval, as shown in FIG. 7. Thus, the ultrasound probe 310 is configured to convert the first Doppler mode transmission signals provided from the transmitting section 320 into the ultrasound signals, transmit the ultrasound signals to the living body in the first transmission direction $Tx_1$, and receive the ultrasound echo signals from the living body to output the first Doppler mode reception signals. The transmitting section 320 is further configured to generate the second Doppler mode transmission signals in consideration of the second transmission direction $Tx_2$ and the elements 311 based on the pulse repeat interval, as shown in FIG. 7. As such, the ultrasound probe 310 is configured to convert the second Doppler mode transmission signals provided from the transmitting section 320 into the ultrasound signals, transmit the ultrasound signals to the living body in the second transmission direction $Tx_2$, and receive the ultrasound echo signals from the living body to output the second Doppler mode reception signals.

As described above, the transmitting section 320 is configured to generate the first Doppler mode transmission signals and the second Doppler mode transmission signals corresponding to the ensemble number based on the pulse repeat interval.

Referring back to FIG. 3, the ultrasound data acquiring unit 120 further includes a receiving section 330. The receiving section 330 is configured to perform an analog-digital conversion upon the reception signals provided from the ultrasound probe 310 to form sampling data of the reception signals. The receiving section 330 is further configured to perform a reception beam-forming upon the sampling data in consideration of the elements 311 to form reception-focused data. The reception beam-forming will be described below in detail.

In one embodiment, the receiving section 330 is configured to perform the analog-digital conversion upon the brightness mode reception signals provided from the ultrasound probe 310 to form sampling data (hereinafter, referred to as "brightness mode sampling data"). The receiving section 330 is further configured to perform the reception beam-forming upon the brightness mode sampling data to form the reception-focused data (hereinafter, referred to as "brightness mode reception-focused data").

The receiving section 330 is further configured to perform the analog-digital conversion upon the Doppler mode reception signals provided from the ultrasound probe 310 to form sampling data (hereinafter, referred to as "Doppler mode sampling data"). The receiving section 330 is also configured to perform the reception beam-forming upon the Doppler mode reception signals to form reception-focused data (hereinafter, referred to as "Doppler mode reception-focused data") corresponding to at least one reception direction of the ultrasound echo signals (i.e., reception beam).

As one example, the receiving section 330 is configured to perform the analog-digital conversion upon the Doppler mode reception signals provided from the ultrasound probe 310 to form the Doppler mode sampling data. The receiving section 330 is further configured to perform the reception beam-forming upon the Doppler mode sampling data to form first Doppler mode reception-focused data corresponding to the first reception direction $Rx_1$ and second Doppler mode reception-focused data corresponding to the second reception direction $Rx_2$, as shown in FIG. 4.

As another example, the receiving section 330 is configured to perform the analog-digital conversion upon the first Doppler mode reception signals provided from the ultrasound probe 310 to form first Doppler mode sampling data corresponding to the first transmission direction $Tx_1$, as shown in FIG. 5. The receiving section 330 is further configured to perform the reception beam-forming upon the first Doppler mode sampling data to form the first Doppler mode reception-focused data corresponding to the first reception direction $Rx_1$. The receiving section 330 is also configured to perform the analog-digital conversion upon the second Doppler mode reception signals provided from the ultrasound probe 310 to form second Doppler mode sampling data corresponding to the second transmission direction $Tx_2$, as shown in FIG. 5. The receiving section 330 is further configured to perform the reception beam-forming upon the second Doppler mode sampling data to form the second Doppler mode reception-focused data corresponding to the second reception direction $Rx_2$. If the reception direction is perpendicular to the elements 311 of the ultrasound probe 310, then an aperture size capable of receiving the ultrasound signals can be a maximum value.

The reception beam-forming may be described with reference to the accompanying drawings.

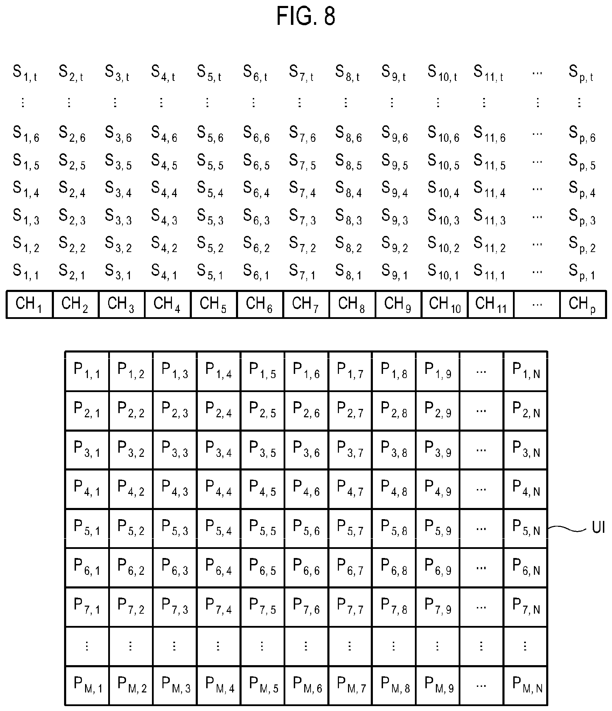

In one embodiment, the receiving section 330 is configured to perform the analog-digital conversion upon the reception signals provided through a plurality of channels $CH_k$, wherein $1 \leq k \leq N$, from the ultrasound probe 310 to form sampling data $S_{i,j}$, wherein the i and j are a positive integer, as shown in FIG. 8. The sampling data $S_{i,j}$ are stored in a storage unit 140.

The receiving section 330 is further configured to detect pixels corresponding to sampling data based on positions of elements 311 and orientation of pixels $P_{a,b}$, wherein $1 \leq a \leq M$, $1 \leq b \leq N$ of the ultrasound image UI with respect to elements 311. That is, the receiving section 330 selects the pixels, which the respective sampling data are used as pixel data thereof, during the reception beam-forming based on the positions of the elements 311 and the orientation of the respective pixels of the ultrasound image UI with respect to the elements 311. The receiving section 330 is configured to cumulatively assign the sampling data corresponding to the selected pixels as pixel data.

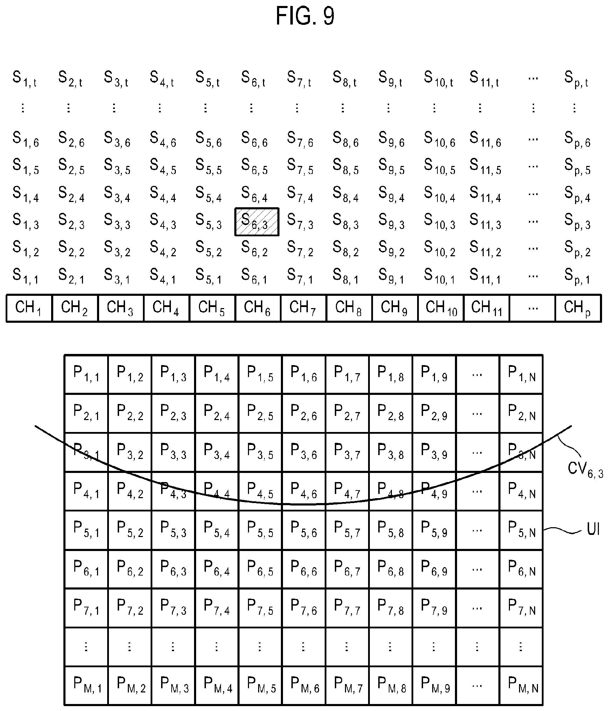

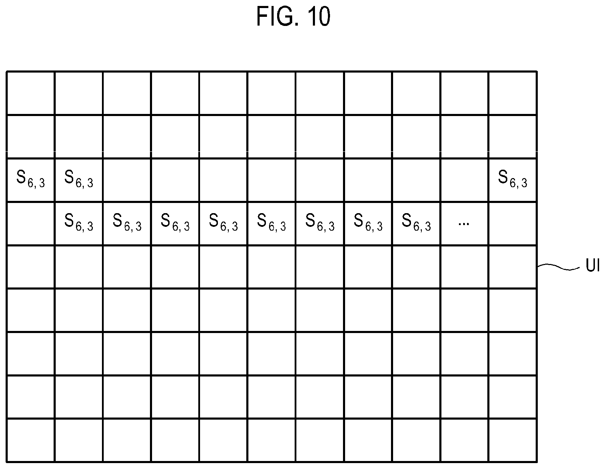

For example, the receiving section 330 is configured to set a curve (hereinafter, referred to as "reception beam-forming curve") $CV_{6,3}$ for selecting pixels, which the sampling data $S_{6,3}$ are used as the pixel data thereof, during the reception beam-forming based on the positions of the elements 311 and the orientation of the respective pixels of the ultrasound image UI with respect to the elements 311, as shown in FIG. 9. The receiving section 330 is further configured to detect the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ corresponding to the reception beam-forming curve $CV_{6,3}$ from the pixels $P_{a,b}$ of the ultrasound image UI. That is, the receiving section 330 selects the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ on which the reception beam-forming curve $CV_{6,3}$ passes among the pixels $P_{a,b}$ of the ultrasound image UI. The receiving section 330 is further configured to assign the sampling data $S_{6,3}$ to the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$, as shown in FIG. 10.

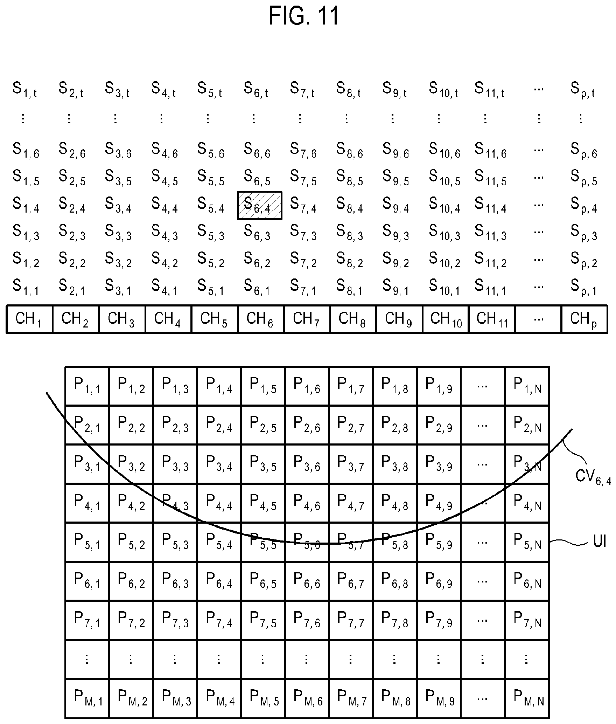

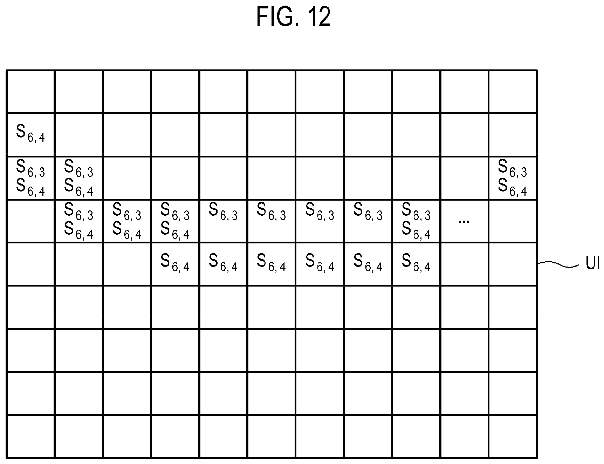

Thereafter, the receiving section 330 is configured to set a reception beam-forming curve $CV_{6,4}$ for selecting pixels, which the sampling data $S_{6,4}$ are used as the pixel data thereof, during the reception beam-forming based on the positions of the elements 311 and the orientation of the respective pixels of the ultrasound image UI with respect to the elements 311, as shown in FIG. 11. The receiving section 330 is further configured to detect the pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$ corresponding to the reception beam-forming curve $CV_{6,4}$ from the pixels $P_{a,b}$ of the ultrasound image UI. That is, the receiving section 330 selects the pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$ on which the reception beam-forming curve $CV_{6,4}$ passes among the pixels $P_{a,b}$ of the ultrasound image UI. The receiving section 330 is further configured to assign the sampling data $S_{6,4}$ to the selected pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$, as shown in FIG. 12. In this way, the respective sampling data, which are used as the pixel data, are cumulatively assigned to the pixels as the pixel data.

The receiving section 330 is configured to perform the reception beam-forming (i.e., summing) upon the sampling data, which are cumulatively assigned to the respective pixels $P_{a,b}$ of the ultrasound image UI to form the reception-focused data.

In another embodiment, the receiving section 330 is configured to perform the analog-digital conversion upon the reception signals provided through the plurality of channels $CH_k$ from the ultrasound probe 310 to form the sampling data $S_{i,j}$, as shown in FIG. 8. The sampling data $S_{i,j}$ are stored in the storage unit 140. The receiving section 330 is further configured to detect pixels corresponding to sampling data based on the positions of the elements 311 and the orientation of the pixels of the ultrasound image UI with respect to the elements 311. That is, the receiving section 330 selects the pixels, which the respective sampling data are used as the pixel data thereof, during the reception beam-forming, based on the positions of the elements 311 and the orientation of the respective pixels of ultrasound image UI with respect to the elements 311. The receiving section 330 is configured to cumulatively assign the sampling data corresponding to the selected pixels as the pixel data. The receiving section 330 is further configured to determine pixels existing in the same column among the selected pixels. The receiving section 330 is further configured to set weights corresponding to the respective determined pixels. The receiving section 330 is also configured to apply the weights to the sampling data of the respective pixels.

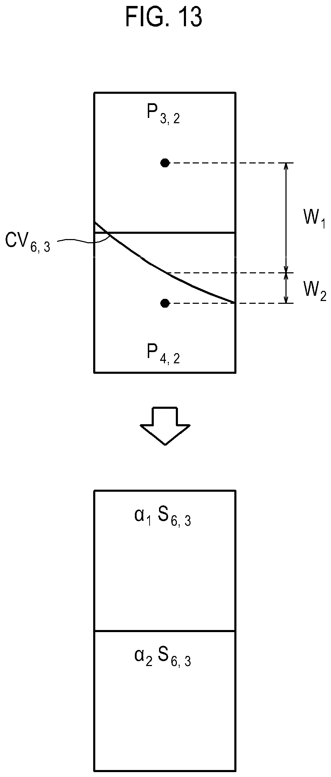

For example, the receiving section 330 is configured to set the reception beam-forming curve $CV_{6,3}$ for selecting pixels, which the sampling data $S_{6,3}$ are used as the pixel data thereof, during the reception beam-forming, based on the positions of the elements 311 and the orientation of the respective pixels of the ultrasound image UI with respect to the elements 311, as shown in FIG. 9. The receiving section 330 is further configured to detect the pixels $P_{3,1}$, $P_{3,2}$, $P_{42}$, $P_{4,3}$, $P_{4,4}$, $P_{4,5}$, $P_{4,6}$, $P_{4,7}$, $P_{4,8}$, $P_{4,9}$, ... $P_{3,N}$ corresponding to the reception beam-forming curve $CV_{6,3}$ from the pixels $P_{a,b}$ of the ultrasound image UI. That is, the receiving section 330 selects the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ on which the reception beam-forming curve $CV_{6,3}$ passes among the pixels $P_{a,b}$ of the ultrasound image UI. The receiving section 330 is also configured to assign the sampling data $S_{6,3}$ to the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$, as shown in FIG. 10. The receiving section 330 is configured to determine pixels $P_{3,2}$ and $P_{4,2}$, which exist in the same column among the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$. The receiving section 330 is further configured to calculate a distance $W_1$ from a center of the determined pixel $P_{3,2}$ to the reception beam-forming curve $CV_{6,3}$ and a distance $W_2$ from a center of the determined pixel $P_{4,2}$ to the reception beam-forming curve $CV_{6,3}$, as shown in FIG. 13. The receiving section 330 is also configured to set a first weight $\alpha_1$ corresponding to the pixel $P_{3,2}$ based on the distance $W_1$ and a second weight $\alpha_2$ corresponding

to the pixel $P_{4,2}$ based on the distance $W_2$. The first weight $\alpha_1$ and the second weight $\alpha_2$ are set to be in proportion to or in inverse proportion to the calculated distances. The receiving section 330 is further configured to apply the first weight $\alpha_1$ to the sampling data $S_{6,3}$ assigned to the pixel $P_{3,2}$ and to apply the second weight $\alpha_2$ to the sampling data $S_{6,3}$ assigned to the pixel $P_{4,2}$. The receiving section 330 is configured to perform the above process upon the remaining sampling data.

The receiving section 330 is configured to perform the reception beam-forming upon the sampling data, which are cumulatively assigned to the respective pixels $P_{a,b}$ of the ultrasound image UI to form the reception-focused data.

In yet another embodiment, the receiving section 330 is configured to perform the analog-digital conversion upon the reception signals provided through the plurality of channels $CH_k$ from the ultrasound probe 310 to form the sampling data $S_{i,j}$, as shown in FIG. 8. The sampling data $S_{i,j}$ are stored in the storage unit 140. The receiving section 330 is further configured to set a sample data set for selecting pixels, which the sampling data $S_{i,j}$ are used as pixel data thereof, during the reception beam-forming.

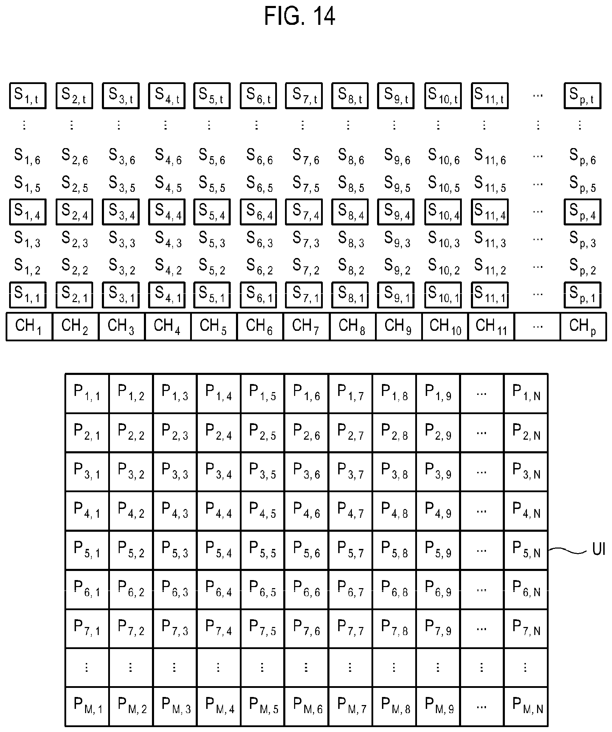

For example, the receiving section 330 is configured to set the sampling data $S_{1,1}$, $S_{1,4}$, ... $S_{1,t}$, $S_{2,1}$, $S_{2,4}$, ... $S_{2,t}$, ... $S_{p,t}$ as the sampling data set (denoted by a box) for selecting the pixels, which the sampling data $S_{i,j}$ are used as the pixel data thereof, during the reception beam-forming, as shown in FIG. 14.

The receiving section 330 is further configured to detect the pixels corresponding to the respective sampling data of the sampling data set based on the positions of the elements 311 and the orientation of the respective pixels of the ultrasound image UI with respect to the elements 311. That is, the receiving section 330 selects the pixels, which the respective sampling data of the sampling data set are used as the pixel data thereof, during the reception beam-forming based on the positions of the elements 311 and the orientation of the respective pixels of the ultrasound image UI with respect to the elements 311. The receiving section 330 is further configured to cumulatively assign the sampling data to the selected pixels in the same manner with the above embodiments. The receiving section 330 is also configured to perform the reception beam-forming upon the sampling data, which are cumulatively assigned to the respective pixels of the ultrasound image UI to form the reception-focused data.

In yet another embodiment, the receiving section 330 is configured to perform a down-sampling upon the reception signals provided through the plurality of channels $CH_k$ from the ultrasound probe 310 to form down-sampled data. As described above, the receiving section 330 is further configured to detect the pixels corresponding to the respective sampling data based on the positions of the elements 311 and the orientation of the respective pixels of the ultrasound image UI with respect to the elements 311. That is, the receiving section 330 selects the pixels, which the respective sampling data are used as the pixel data thereof, during the reception beam-forming based on the positions of the elements 311 and the orientation of the pixels of the ultrasound image UI with respect to the elements 311. The receiving section 330 is further configured to cumulatively assign the respective sampling data to the selected pixels in the same manner as the above embodiments. The receiving section 330 is also configured to perform the reception beam-forming upon the sampling data, which are cumulatively assigned to the respective pixels of the ultrasound image UI to form the reception-focused data.

However it should be noted herein that the reception beam-forming may not be limited thereto.

Referring back to FIG. 3, the ultrasound data acquiring unit 120 further includes an ultrasound data forming section 340. The ultrasound data forming section 340 is configured to form the ultrasound data corresponding to the ultrasound image based on the reception-focused data provided from the receiving section 330. The ultrasound data forming section 340 is also configured to perform a signal process (e.g., gain control, etc.) upon the reception-focused data.

In one embodiment, the ultrasound data forming section 340 is configured to form ultrasound data (hereinafter, referred to as "brightness mode ultrasound data") corresponding to the brightness mode image based on the brightness mode reception-focused data provided from the receiving section 330. The brightness mode ultrasound data include radio frequency data. However, it should be noted herein that the brightness mode ultrasound data may not be limited thereto.

The ultrasound data forming section 340 is further configured to form ultrasound data (hereinafter, referred to as "Doppler mode ultrasound data") corresponding the region of interest ROI based on the Doppler mode reception-focused data provided from the receiving section 330. The Doppler mode ultrasound data include in-phase/quadrature data. However, it should be noted herein that the Doppler mode ultrasound data may not be limited thereto.

For example, the ultrasound data forming section 340 forms first Doppler mode ultrasound data based on the first Doppler mode reception-focused data provided from the receiving section 330. The ultrasound data forming section 340 further forms second Doppler mode ultrasound data based on the second Doppler mode reception-focused data provided from the receiving section 330.

Referring back to FIG. 1, the ultrasound system 100 also includes a processing unit 130 in communication with the user input unit 110 and the ultrasound data acquiring unit 120. The processing unit 130 includes a central processing unit, a microprocessor, a graphic processing unit and the like.

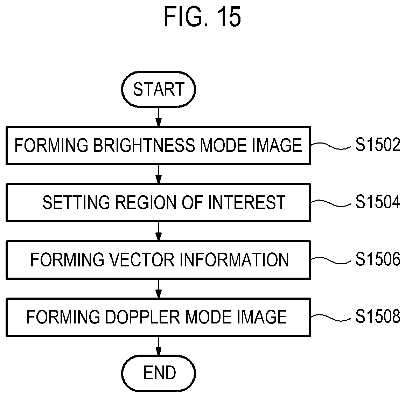

FIG. 15 is a flow chart showing a process of forming the vector information based on the vector Doppler. The processing unit 130 is configured to the brightness mode image BI based on the brightness mode ultrasound data provided from the ultrasound data acquiring unit 120, at step S1502 in FIG. 15. The brightness mode image BI is displayed on a display unit 150. Thus, the user sets the region of interest ROI on the Brightness mode image BI displayed on the display unit 150 by using the user input unit 110.

The processing unit 130 is configured to set the region of interest ROI on the brightness mode image BI based on the input information provided from the user input unit 110, at step S1504 in FIG. 15. Thus, the ultrasound data acquiring unit 120 transmits the ultrasound signals to the living body and receives the ultrasound echo signals from the living body to acquire the Doppler mode ultrasound data, in consideration of the region of interest ROI.

The processing unit 130 is configured to form the vector information based on the Doppler mode ultrasound data provided from the ultrasound data acquiring unit 120, at step S1506 in FIG. 15. That is, the processing unit 130 detects the vector information corresponding to motion (i.e., velocity and direction) of the target object based on the Doppler mode ultrasound data.

Generally, when the transmission direction of the ultrasound signals is equal to the reception direction of the ultrasound echo signals and a Doppler angle is $\theta$, the following relationship is established:

$$X \cos \theta = \frac{C_0 f_d}{2 f_0} \qquad (1)$$

In equation 1, X represents a reflector velocity (i.e., velocity of blood flow), $C_0$ represents a sound speed in the living body, $f_d$ represents a Doppler shift frequency, and $f_0$ represents an ultrasound frequency.

The Doppler shift frequency $f_d$ is calculated by the difference between a frequency of the ultrasound signals (i.e., transmission beam) and a frequency of the ultrasound echo signals (i.e., reception beam). Also, the velocity component $X\cos\theta$ projected to the transmission direction is calculated by equation 1.

When the transmission direction of the ultrasound signals (i.e., transmission beam) is different to the reception direction of the ultrasound echo signals (i.e., reception beam), the following relationship is established:

$$X \cos \theta_T + X \cos \theta_R = \frac{C_0 f_d}{f_0} \qquad (2)$$

In equation 2, $\theta_T$ represents an angle between the ultrasound signals (i.e., transmission beam) and the blood flow, and $\theta_R$ represents an angle between the ultrasound echo signals (i.e., reception beam) and the blood flow.

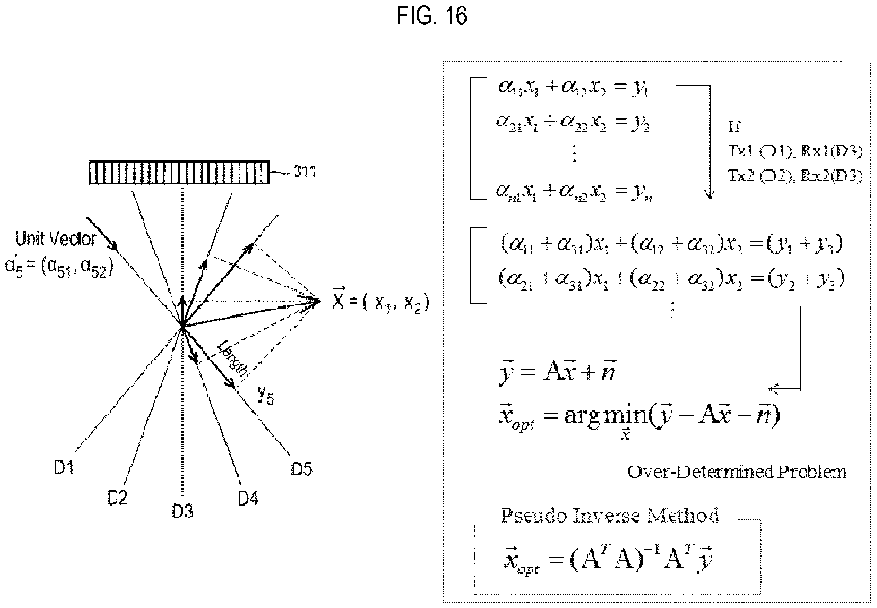

FIG. 16 is a schematic diagram showing an example of the transmission directions, the reception directions, the vector information and an over-determined problem. Referring to FIG. 16, when the ultrasound signals (i.e., transmission beam) are transmitted in a first direction D1 and the ultrasound echo signals (i.e., reception beam) are received in the first direction D1, the following relationship is established:

$$\overrightarrow{\alpha_1} \overrightarrow{X} = \alpha_{11} x_1 + \alpha_{12} x_2 = y_1 = X \cos \theta \qquad (3)$$

In equation 3, $\overrightarrow{\alpha_1} = (\alpha_{11}, \alpha_{12})$ represents a unit vector of the first direction D1, $\overrightarrow{X} = (x_1, x_2)$ represents variables, and $y_1$ is calculated by the equation 1.

When the ultrasound signals (i.e., transmission beam) are transmitted in a second direction D2 and the ultrasound echo signals (i.e., reception beam) are received in a third direction D3, the following relationship is established:

$$(\alpha_{21} + \alpha_{31}) x_1 + (\alpha_{22} + \alpha_{32}) x_2 = (y_2 + y_3) = X \cos \theta_2 + X \cos \theta_3 \qquad (4)$$

Equations 3 and 4 assume a two-dimensional environment, wherein equations 3 and 4 may be expanded to a three-dimensional environment. That is, when expanding equations 3 and 4 to the three-dimensional environment, the following relationship is established:

$$\alpha_{11} x_1 + \alpha_{12} x_2 + \alpha_{13} x_3 = y \qquad (5)$$

In the case of the two-dimensional environment (i.e., two-dimensional vector), at least two equations are required to calculate the variables $x_1$ and $x_2$. For example, when the ultrasound signals (i.e., transmission beam) are transmitted in the third direction D3 and the ultrasound echo signals (i.e., reception beam) are received in the second direction D2 and a fourth direction D4 as shown in FIG. 16, the following equations is established:

$$(\alpha_{31} + \alpha_{21})x_1 + (\alpha_{32} + \alpha_{22})x_2 = (y_3 + y_2)$$

$$(\alpha_{31} + \alpha_{41})x_1 + (\alpha_{32} + \alpha_{42})x_2 = (y_3 + y_4) \qquad (6)$$

The vector $\vec{X} = (x_1, x_2)$ is calculated by the two equations of equation 6.

When the reception beam-forming is performed in at least two angles (i.e., at least two reception directions), at least two equations are obtained and represented as the over-determined problem, as shown in FIG. 16. The over-determined problem is solved by a pseudo inverse method, a weighted least square method and the like based on noise characteristics added to the Doppler shift frequency. The over-determined problem is well known in the art. Thus, it will not be described in detail so as not to unnecessarily obscure the present disclosure. That is, MxN equations are obtained by M transmission directions and the reception beam-forming of N reception directions at every transmission. Optionally, the processing unit 130 is configured to form a Doppler mode image based on the vector information, at step S1508 in FIG. 15. The Doppler mode image includes a vector Doppler image or a color Doppler image. However, it should be noted herein that the Doppler mode image may not be limited thereto. The methods of forming the Doppler mode image based on the vector information are well known in the art. Thus, they have not been described in detail so as not to unnecessarily obscure the present disclosure.

The processing unit 130 is also configured to perform an image compound process upon the brightness mode image and the Doppler mode image to form a compound image.

Referring back to FIG. 1, the ultrasound system 100 further includes the storage unit 140. The storage unit 140 stores the ultrasound data (i.e., brightness mode ultrasound data and Doppler mode ultrasound data) acquired by the ultrasound data acquiring unit 120. The storage unit 140 further stores the vector information formed by the processing unit 130.

The ultrasound system 100 further includes the display unit 150. The display unit 150 is configured to display the brightness mode image BI formed by the processing unit 130. The display unit 150 is further configured to display the Doppler mode image formed by the processing unit 130.

## Claims

1. An ultrasound system (100), comprising:

   an ultrasound data acquiring unit (120) configured to transmit a plurality of ultrasound signals to a moving target object included in a living body in M transmission directions,
   and receive a plurality of ultrasound echo signals from the moving target object included in the living body in N reception
   directions for each and every transmission of the plurality of ultrasound signals to acquire ultrasound data; and
   a processing unit (130) configured to form vector information corresponding to a motion of the moving target object based on the ultrasound data, by defining an over-determined
   system of M x N equations obtained according to the M transmission directions and the N reception directions, and by solving the over-determined system of M x N equations by using a pseudo inverse method or a weighted least squares method to form the vector information corresponding to the motion of the moving target object, wherein the M and the N are integer numbers bigger than 1.

2. The ultrasound system (100) of claim 1, wherein the ultrasound data acquiring unit (120) is configured to transmit the plurality of ultrasound signals in an interleaved transmission scheme.

3. The ultrasound system (100) of claim 1, wherein the plurality of ultrasound signals include plane wave signals or focused signals.

4. The ultrasound system (100) of claim 1, wherein the M transmission directions and the N reception directions are directions in a three-dimensional environment, and the vector information formed by defining and solving the over-determined system of M x N equations contain three components indicating directions in the three-dimensional environment.

**5.** A method of forming vector information, comprising:

a) transmitting a plurality of ultrasound signals to a moving target object included in a living body in M transmission directions, and receiving a plurality of ultrasound echo signals from the moving target object included in the living body in N reception directions to acquire ultrasound data for each and every transmission of the plurality of ultrasound signals; and

b) forming vector information corresponding to a motion of the moving target object based on the ultrasound data, wherein the step b) comprises:

defining an over-determined system of M x N equations obtained according to the M transmission directions and the N reception directions, and

solving the over-determined system of M x N equations by using a pseudo inverse method or a weighted least squares method to form the vector information corresponding to the motion of the moving target object, wherein the M and the N are integer numbers bigger than 1.

**6.** The method of claim 5, wherein the plurality of ultrasound signals are transmitted in an interleaved transmission scheme.

**7.** The method of claim 5, wherein the plurality of ultrasound signals include plane wave signals or focused signals.

**8.** The method of claim 5, wherein M transmission directions and the N reception directions are directions in a three-dimensional environment, and the vector information formed by defining and solving the over-determined system of M x N equations contain three components indicating directions in the three-dimensional environment.

**Patentansprüche**

**1.** Ultraschallsystem (100), welches Folgendes aufweist:

eine Ultraschalldatenerlangungseinheit (120), die dafür vorgesehen ist, eine Vielzahl von Ultraschallsignalen zu einem sich bewegenden Zielobjekt, das in einem lebenden Körper enthalten ist, in M Übertragungsrichtungen zu übertragen, und eine Vielzahl von Ultraschallechosignalen von dem sich bewegenden Zielobjekt, das in dem lebenden Körper enthalten ist, in N Empfangsrichtungen für jede einzelne Übertragung der Vielzahl von Ultraschallsignalen zu empfangen, um Ultraschalldaten zu erlangen; und

eine Verarbeitungseinheit (130), die dafür vorgesehen ist, Vektorinformationen, die mit einer Bewegung des sich bewegenden Zielobjekts korrespondieren, basierend auf den Ultraschallsignalen durch Definieren eines überbestimmten Systems von M x N Gleichungen, die gemäß den M Übertragungsrichtungen und den N Empfangsrichtungen erlangt worden sind, und durch Lösen des überbestimmten Systems der M x N Gleichungen unter Verwendung eines pseudoinversen Verfahrens oder eines Verfahrens der gewichteten kleinsten Quadrate zu erzeugen, um die Vektorinformationen entsprechend der Bewegung des sich bewegenden Zielobjekts zu erzeugen,

wobei das M und das N ganze Zahlen größer als 1 sind.

**2.** Ultraschallsystem (100) nach Anspruch 1, wobei die Ultraschalldatenerlangungseinheit (120) dafür vorgesehen ist, die Vielzahl von Ultraschallsignalen in einem Zwischensendungsschema zu übertragen.

**3.** Ultraschallsystem (100) nach Anspruch 1, wobei die Vielzahl von Ultraschallsignalen ebene Wellensignale oder fokussierte Signale beinhalten.

**4.** Ultraschallsystem (100) nach Anspruch 1, wobei die M Übertragungsrichtungen und die N Empfangsrichtungen Richtungen in einer dreidimensionalen Umgebung sind, und wobei die Vektorinformationen, die durch Definieren und Lösen des überbestimmten Systems von M x N Gleichungen gebildet wurden, drei Komponenten beinhalten, die Richtungen in der dreidimensionalen Umgebung anzeigen.

**5.** Verfahren zum Erzeugen von Vektorinformationen, welches Folgendes aufweist:

a) Übertragen einer Vielzahl von Ultraschallsignalen zu einem sich bewegenden Zielobjekt, das in einem lebenden Körper enthalten ist, in M Übertragungsrichtungen und Empfangen einer Vielzahl von Ultraschallechosignalen von dem sich bewegenden Zielobjekt, das in dem lebenden Körper enthalten ist, in N Empfangsrichtungen, um Ultraschalldaten für jede einzelne Übertragung der Vielzahl von Ultraschallsignalen zu erlangen; und
b) Erzeugen von Vektorinformationen, die mit einer Bewegung des sich bewegenden Zielobjekts basierend auf den Ultraschalldaten korrespondiert,
wobei der Schritt b) Folgendes aufweist:

Definieren eines überbestimmten Systems von M x N Gleichungen, die gemäß den M Übertragungsrichtungen und den N Empfangsrichtungen erlangt wurden, und Lösen des überbestimmten Systems von M x N Gleichungen durch Verwenden eines pseudoinversen Verfahrens oder eines Verfahrens der gewichteten kleinsten Quadrate, um die Vektorinformationen, die mit der Bewegung des sich bewegenden Zielobjekts korrespondieren, zu erzeugen,
wobei das M und das N ganze Zahlen größer als 1 sind.

**6.** Verfahren nach Anspruch 5, wobei die Vielzahl von Ultraschallsignalen in einem Zwischensendungsschema übertragen werden.

**7.** Verfahren nach Anspruch 5, wobei die Vielzahl von Ultraschallsignalen ebene Wellensignale oder fokussierte Signale beinhalten.

**8.** Verfahren nach Anspruch 5, wobei die M Übertragungsrichtungen und die N Empfangsrichtungen Richtungen in einer dreidimensionalen Umgebung sind, und wobei die Vektorinformationen, die durch Definieren und Lösen des überbestimmten Systems von M x N Gleichungen gebildet wurden, drei Komponenten beinhalten, die Richtungen in der dreidimensionalen Umgebung anzeigen.

**Revendications**

**1.** Système à ultrasons (100) comprenant :

une unité d'acquisition de données ultrasonores (120) configurée pour transmettre une pluralité de signaux ultrasonores à un objet cible en mouvement inclus dans un corps vivant dans M directions de transmission, et recevoir une pluralité de signaux d'écho ultrasonores depuis l'objet cible en mouvement inclus dans le corps vivant dans N directions de réception pour chaque transmission de la pluralité de signaux ultrasonores afin d'acquérir des données ultrasonores ; et
une unité de traitement (130) configurée pour former des informations vectorielles correspondant à un mouvement de l'objet cible en mouvement sur la base des données ultrasonores, en définissant un système surdéterminé de M x N équations obtenues selon les M directions de transmission et les N directions de réception, et en résolvant le système surdéterminé de M x N équations en utilisant une méthode pseudo inverse ou une méthode des moindres carrés pondérés pour former les informations vectorielles correspondant au mouvement de l'objet cible en mouvement,
dans lequel M et N sont des nombres entiers supérieurs à 1.

**2.** Système à ultrasons (100) selon la revendication 1, dans lequel l'unité d'acquisition de données ultrasonores (120) est configurée pour transmettre la pluralité de signaux ultrasonores dans un schéma de transmission entrelacée.

**3.** Système à ultrasons (100) selon la revendication 1, dans lequel la pluralité de signaux ultrasonores comprend des signaux à ondes planes ou des signaux focalisés.

**4.** Système à ultrasons (100) selon la revendication 1, dans lequel les M directions de transmission et les N directions de réception sont des directions dans un environnement tridimensionnel, et les informations vectorielles formées en définissant et en résolvant le système surdéterminé de M x N équations contiennent trois composantes indiquant des directions dans l'environnement tridimensionnel.

**5.** Procédé de formation d'informations vectorielles, comprenant les étapes consistant à :

a) transmettre une pluralité de signaux ultrasonores à un objet cible en mouvement inclus dans un corps vivant dans M directions de transmission, et recevoir une pluralité de signaux d'écho ultrasonores depuis l'objet cible en mouvement inclus dans le corps vivant dans N directions de réception pour chaque transmission de la pluralité de signaux ultrasonores ; et

b) former des informations vectorielles correspondant à un mouvement de l'objet cible en mouvement sur la base des données ultrasonores, dans lequel l'étape b) comprend les étapes consistant à :

définir un système surdéterminé de M x N équations obtenues selon les M directions d'émission et les N directions de réception, et

résoudre le système surdéterminé de M x N équations en utilisant une méthode pseudo inverse ou une méthode des moindres carrés pondérés pour former les informations vectorielles correspondant au mouvement de l'objet cible en mouvement, dans lequel M et N sont des nombres entiers supérieurs à 1.

6. Procédé selon la revendication 5, dans lequel la pluralité de signaux ultrasonores sont transmis dans un schéma de transmission entrelacée.

7. Procédé selon la revendication 5, dans lequel la pluralité de signaux ultrasonores comprend des signaux à ondes planes ou des signaux focalisés.

8. Procédé selon la revendication 5, dans lequel les M directions de transmission et les N directions de réception sont des directions dans un environnement tridimensionnel, et les informations vectorielles formées en définissant et en résolvant le système surdéterminé de M x N équations contiennent trois composantes indiquant des directions dans l'environnement tridimensionnel.

FIG. 1

<u>100</u>

110

USER
INPUT UNIT

120

ULTRASOUND
DATA ACQUIRING
UNIT

130

PROCESSING
UNIT

150

DISPLAY
UNIT

140

STORAGE
UNIT

# FIG. 2

# FIG. 3

# FIG. 4

311

Rx₂    Rx₁

Tx

## FIG. 5

# FIG. 6

# FIG. 7

311

Rx₁          Rx₂

Tx₁          Tx₂

Tx₁  PRI  Tx₂  PRI  Tx₁  PRI  Tx₂  ...

time

# FIG. 8

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ | |
|---|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ | |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ | |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ | |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ | $\sim$ UI |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ | |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ | |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ | |

# FIG. 9

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
| --- | --- | --- | --- | --- | --- | --- | --- | --- | --- | --- | --- | --- |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
| --- | --- | --- | --- | --- | --- | --- | --- | --- | --- | --- |
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

$CV_{6,3}$

UI

FIG. 10

| | | | | | | | | | | |
|---|---|---|---|---|---|---|---|---|---|---|
| | | | | | | | | | | |
| | | | | | | | | | | |
| $S_{6,3}$ | $S_{6,3}$ | | | | | | | | | $S_{6,3}$ |
| | $S_{6,3}$ | $S_{6,3}$ | $S_{6,3}$ | $S_{6,3}$ | $S_{6,3}$ | $S_{6,3}$ | $S_{6,3}$ | $S_{6,3}$ | ... | |
| | | | | | | | | | | |
| | | | | | | | | | | |
| | | | | | | | | | | |
| | | | | | | | | | | |
| | | | | | | | | | | |

UI

# FIG. 11

$S_{1,t}$   $S_{2,t}$   $S_{3,t}$   $S_{4,t}$   $S_{5,t}$   $S_{6,t}$   $S_{7,t}$   $S_{8,t}$   $S_{9,t}$   $S_{10,t}$   $S_{11,t}$   $\cdots$   $S_{p,t}$

$\vdots$    $\vdots$    $\vdots$    $\vdots$    $\vdots$    $\vdots$    $\vdots$    $\vdots$    $\vdots$    $\vdots$    $\vdots$    $\vdots$    $\vdots$

$S_{1,6}$   $S_{2,6}$   $S_{3,6}$   $S_{4,6}$   $S_{5,6}$   $S_{6,6}$   $S_{7,6}$   $S_{8,6}$   $S_{9,6}$   $S_{10,6}$   $S_{11,6}$   $\cdots$   $S_{p,6}$

$S_{1,5}$   $S_{2,5}$   $S_{3,5}$   $S_{4,5}$   $S_{5,5}$   $S_{6,5}$   $S_{7,5}$   $S_{8,5}$   $S_{9,5}$   $S_{10,5}$   $S_{11,5}$   $\cdots$   $S_{p,5}$

$S_{1,4}$   $S_{2,4}$   $S_{3,4}$   $S_{4,4}$   $S_{5,4}$   $\boxed{S_{6,4}}$   $S_{7,4}$   $S_{8,4}$   $S_{9,4}$   $S_{10,4}$   $S_{11,4}$   $\cdots$   $S_{p,4}$

$S_{1,3}$   $S_{2,3}$   $S_{3,3}$   $S_{4,3}$   $S_{5,3}$   $S_{6,3}$   $S_{7,3}$   $S_{8,3}$   $S_{9,3}$   $S_{10,3}$   $S_{11,3}$   $\cdots$   $S_{p,3}$

$S_{1,2}$   $S_{2,2}$   $S_{3,2}$   $S_{4,2}$   $S_{5,2}$   $S_{6,2}$   $S_{7,2}$   $S_{8,2}$   $S_{9,2}$   $S_{10,2}$   $S_{11,2}$   $\cdots$   $S_{p,2}$

$S_{1,1}$   $S_{2,1}$   $S_{3,1}$   $S_{4,1}$   $S_{5,1}$   $S_{6,1}$   $S_{7,1}$   $S_{8,1}$   $S_{9,1}$   $S_{10,1}$   $S_{11,1}$   $\cdots$   $S_{p,1}$

| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

$CV_{6,4}$

UI

FIG. 12

| | | | | | | | | | | |
|---|---|---|---|---|---|---|---|---|---|---|
| $S_{6,4}$ | | | | | | | | | | |
| $S_{6,3}$ $S_{6,4}$ | $S_{6,3}$ $S_{6,4}$ | | | | | | | | $S_{6,3}$ $S_{6,4}$ | |
| | $S_{6,3}$ $S_{6,4}$ | $S_{6,3}$ $S_{6,4}$ | $S_{6,3}$ $S_{6,4}$ | $S_{6,3}$ | $S_{6,3}$ | $S_{6,3}$ | $S_{6,3}$ | $S_{6,3}$ $S_{6,4}$ | ... | |
| | | | $S_{6,4}$ | $S_{6,4}$ | $S_{6,4}$ | $S_{6,4}$ | $S_{6,4}$ | $S_{6,4}$ | | |
| | | | | | | | | | | |
| | | | | | | | | | | |
| | | | | | | | | | | |
| | | | | | | | | | | |

UI

# FIG. 13

# FIG. 14

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |

$\vdots$　$\vdots$　$\vdots$　$\vdots$　$\vdots$　$\vdots$　$\vdots$　$\vdots$　$\vdots$　$\vdots$　$\vdots$　$\vdots$　$\vdots$

$S_{1,6}$　$S_{2,6}$　$S_{3,6}$　$S_{4,6}$　$S_{5,6}$　$S_{6,6}$　$S_{7,6}$　$S_{8,6}$　$S_{9,6}$　$S_{10,6}$　$S_{11,6}$　$\cdots$　$S_{p,6}$

$S_{1,5}$　$S_{2,5}$　$S_{3,5}$　$S_{4,5}$　$S_{5,5}$　$S_{6,5}$　$S_{7,5}$　$S_{8,5}$　$S_{9,5}$　$S_{10,5}$　$S_{11,5}$　$\cdots$　$S_{p,5}$

| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |

$S_{1,3}$　$S_{2,3}$　$S_{3,3}$　$S_{4,3}$　$S_{5,3}$　$S_{6,3}$　$S_{7,3}$　$S_{8,3}$　$S_{9,3}$　$S_{10,3}$　$S_{11,3}$　$\cdots$　$S_{p,3}$

$S_{1,2}$　$S_{2,2}$　$S_{3,2}$　$S_{4,2}$　$S_{5,2}$　$S_{6,2}$　$S_{7,2}$　$S_{8,2}$　$S_{9,2}$　$S_{10,2}$　$S_{11,2}$　$\cdots$　$S_{p,2}$

| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

UI

FIG. 15

```
                    ┌─────────┐
                    │  START  │
                    └────┬────┘
                         ↓
    ┌────────────────────────────────────────┐
    │     FORMING BRIGHTNESS MODE IMAGE       ├──── S1502
    └────────────────────┬───────────────────┘
                         ↓
    ┌────────────────────────────────────────┐
    │        SETTING REGION OF INTEREST       ├──── S1504
    └────────────────────┬───────────────────┘
                         ↓
    ┌────────────────────────────────────────┐
    │       FORMING VECTOR INFORMATION        ├──── S1506
    └────────────────────┬───────────────────┘
                         ↓
    ┌────────────────────────────────────────┐
    │      FORMING DOPPLER MODE IMAGE         ├──── S1508
    └────────────────────┬───────────────────┘
                         ↓
                    ┌─────────┐
                    │   END   │
                    └─────────┘
```

FIG. 16

$$\begin{bmatrix} \alpha_{11}x_1 + \alpha_{12}x_2 = y_1 \\ \alpha_{21}x_1 + \alpha_{22}x_2 = y_2 \\ \vdots \\ \alpha_{n1}x_1 + \alpha_{n2}x_2 = y_n \end{bmatrix}$$

If
Tx1 (D1), Rx1(D3)
Tx2 (D2), Rx2(D3)

$$\begin{bmatrix} (\alpha_{11} + \alpha_{31})x_1 + (\alpha_{12} + \alpha_{32})x_2 = (y_1 + y_3) \\ (\alpha_{21} + \alpha_{31})x_1 + (\alpha_{22} + \alpha_{32})x_2 = (y_2 + y_3) \\ \vdots \end{bmatrix}$$

$$\vec{y} = A\vec{x} + \vec{n}$$

$$\vec{x}_{opt} = \arg\min_{\vec{x}}(\vec{y} - A\vec{x} - \vec{n})$$

Over-Determined Problem

Pseudo Inverse Method

$$\vec{x}_{opt} = (A^T A)^{-1} A^T \vec{y}$$

Unit Vector
$\vec{a}_5 = (a_{51}, a_{52})$

311

$\vec{X} = ( x_1 , x_2 )$

Length

$y_5$

D1
D2
D3
D4
D5

EP 2 610 638 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020110143861

### Non-patent literature cited in the description

- **DUNMIRE B. et al.** Cross-beam vector Doppler ultrasound for angle-independent velocity measurements. *ULTRASOUND IN MEDICINE AND BIOLOGY,* 01 October 2000, vol. 26, ISSN 0301-5629, 1213-1235
- Diffractive transducers for angle-independent velocity measurements. **VILKOMERSON D. et al.** ULTRASONICS SYMPOSIUM, 1994. PROCEEDINGS., 1994 IEEE Cannes. IEEE, 31 October 1994, 1677
- 2-D high-frame-rate dynamic elastography using delay compensated and angularly compounded motion vectors: Preliminary results. **REZA ZAHIRI AZAR et al.** IEEE TRANSACTIONS ON ULTRASONICS, FERROELECTRICS AND FREQUENCY CONTROL. IEEE, 01 November 2010, vol. 57, 2421-2436